# EUROPEAN PATENT APPLICATION

(11) **EP 2 030 631 A1**
(43) Date of publication of application: **04.03.2009**
(21) Application number: 07017091.5
(22) Date of filing: 31.08.2007
(51) Int. Cl.: A61K 48/00, A61K 38/17

(54) **Means and methods for treating peripheral and cardiovascular diseases via modulation of arteriogenesis**

(71) Applicant: Max-Planck-Gesellschaft zur Förderung der Wissenschaften e.V., 80539 München (DE)
(72) Inventor: Schaper, Wolfgang, 61231 Bad Nauheim (DE); Troidl, Kerstin, 61231 Bad Nauheim (DE); Troidl, Christian, 61231 Bad Nauheim (DE); Eitenmüller, Inka K., 64753 Brombachtal (DE)
(74) Representative: Vossius & Partner

(57) **Abstract**

The present invention relates to a pharmaceutical composition comprising (a) a polynucleotide comprising or consisting of a nucleotide sequence encoding a polypeptide with the amino acid sequence as set forth in SEQ ID NO: 2; (b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity; (c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or (d) a polypeptide encoded by the polynucleotide of any one of (a) to (c).

## Description

This invention relates to a pharmaceutical composition comprising (a) a polynucleotide comprising or consisting of a nucleotide sequence encoding a polypeptide with the amino acid sequence as set forth in SEQ ID NO: 2; (b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity; (c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or (d) a polypeptide encoded by the polynucleotide of any one of (a) to (c).

In this specification, a number of documents including patent applications and manufacturer's manuals is cited. The disclosure of these documents, while not considered relevant for the patentability of this invention, is herewith incorporated by reference in its entirety.

Occlusion of an artery as a complication of underlying atherosclerosis is the major cause of death worldwide. Formerly restricted to the industrialized countries, nowadays emerging countries and those of the third world suffer almost equally from organ manifestations of arterial degeneration and occlusion, affecting hearts, brains, kidneys and the limb circulation. Since only a minority of the affected population dies at the first manifestation of the disease, a long and expensive suffering is the result.

Atherosclerosis can be prevented and, when manifest, treated. However, prevention means that many people may have to abandon a life style that they recognize as a hard won status symbol. The drastic rise of obesity in Western countries, even in those with the "healthy Mediterranean diet", is a good example of the near futility of recommended lifestyle changes.

Drug therapy can be effective in reducing blood cholesterol levels, but only 25% of the treated population is spared a heart attack.

Heart surgery and catheter-based re-canalizations of arteries are very effective but very expensive and especially stent-based therapies are still prone to re-occlusion.

Angiogenic growth factors had inspired high hopes but clinical trials had been very disappointing. Growth factors are known to stimulate the sprouting of capillaries but capillaries are unable to replace an occluded artery.

The new stem cell based therapies are as yet extremely controversial and recent Skandinavian clinical studies showed them to be inactive in patients with acute myocardial infarction.

During the evolution of the human race, especially when the need arose to walk and run upright, the healing of leg wounds and the replacement of damaged leg arteries became life saving and so a special mechanism for arterial regeneration was developed from which we still benefit: the establishment of a collateral circulation, the formation of "bypass"-vessels in cases of occlusion of the large arteries. This process, which we call now "arteriogenesis", is operative in the entire arterial system but not as well developed as in the limbs.

Already in the 1970ies it was shown (Schaper et al. (1972), Schaper (1971)) that an arterial occlusion activates the small pre-existent bypass vessels to grow at a very rapid rate: Within one week they were able to furnish normal blood flow to the heart in experimental animals, provided the arterial occlusion proceeded gradually and not abruptly. A limb artery can be acutely occluded but it would take about a week to be able to (slowly) walk again. The process of bypass enlargement is by cell division.

Small bypass vessels can increase their diameter by a factor of 20 and their tissue mass by a factor of 50. The growth process recruits helper cells from the bone marrow, mainly monocytes but also T-lymphocytes. These cells are necessary to break down the old vascular structure to create the space for the much larger new vessel.

In view of the limitations of the methods described in the prior art, the technical problem underlying the present invention was the provision of alternative or improved means and methods for treating ischemic conditions and restenosis.

Accordingly, this invention relates to a pharmaceutical composition comprising (a) a polynucleotide comprising or consisting of a nucleotide sequence encoding a polypeptide with the amino acid sequence as set forth in SEQ ID NO: 2; (b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity; (c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or (d) a polypeptide encoded by the polynucleotide of any one of (a) to (c).

The term "arteriogenic" as used herein means "causative of arteriogenesis" or "having a stimulatory effect on arteriogenesis".

The term "arteriogenesis" as used herein relates to the development of arteries from previously existing collateral arterial vessels. The term "collateral arterial vessels" designates arterial vessels which are secondary or subordinate as compared to the principal arterial vessel in a given part of the human or animal body. The terms "secondary" and "subordinate" relate inter alia to the diameter of and the flow rate (amount of blood per unit of time) through the collateral arterial vessels. The process of arteriogenesis leads to a conversion of one or more of said collateral arterial vessels into the main arterial vessel(s) in said part of the body. The conversion comprises an increase in diameter and a concomitant increase in blood flow rate. The increase in diameter may be 2-, 3-, 4-, 5-, 10- or 20-fold or larger. Concomitantly, the tissue mass of the vessel may increase by a factor of 5, 10, 20 or 50 or above. As a consequence, the converted collateral arterial vessel(s) may serve as a replacement for a damaged or non-functional arterial vessel. In other words, arteriogenesis leads to the formation of bypass vessels.

Arteriogenic activity according to the invention may be assayed by methods known in the art (Schaper et al. (1969), Schaper et al. (1971), Pasyk et al. (1982), Schaper et al. (1976)) and as described in the Examples enclosed herewith, including hemodynamic measurements and angiography such as post mortem angiography.

Hemodynamic measurements permit the determination of maximum collateral conductances. The term "maximum collateral conductance" designates the maximum capacity of collateral circulation. The measurements may comprise systemic pressure measurements, for example in the right carotic artery, and/or peripheral pressure measurements, for example in both saphenous arteries. Synchronously, hindlimb blood flow (e.g. in a rabbit model system) may be detected at increasing adenosine concentrations (100 to 600 µg per kg body weight and per min) in both iliac arteries.

Post mortem angiography may be performed as follows (the example relates to the rabbit model system): Hind limbs of euthanized rabbits are perfused with a gelatine-barium-based contract medium. Angiograms of the hind limbs are taken in a Balteau radiography apparatus. Angiographically visible collateral arteries, spanning from the arteriae profundae femoralis and circumflexa femoris lateralis to the arteriae genualis descendens and caudalis femoris are counted.

The term "arterial" as used herein means "pertaining to an artery or to the arteries". Any type of artery is envisaged. The term "artery" includes aorta (and parts thereof such as ascending aorta, descending aorta, thoracic aorta, abdominal aorta), pulmonary artery, carotis artery (external and internal), arteries of the brain, subclavian artery, axillary artery, brachial artery, radial artery, ulnar artery, hypogastric artery, hepatic artery, iliac artery, femoral artery, popliteal artery, anterior and posterior tibial artery, and arteria dorsalis pedis.

The term "arteriogenesis" is to be distinguished from "angiogenesis". Angiogenesis relates to the formation of new arterial vessels in a region where previously no or less arterial vessels existed. As such it is different from "arteriogenesis" which designates a conversion of smaller into larger arterial vessels.

The sequence of SEQ ID NO: 2 is the sequence of the human TRPV4 protein. The symbol TRPV4 stands for "transient receptor potential cation channel, subfamily V, member 4". TRPV4 is a member of the OSM9-like transient receptor potential channel (OTRPC) subfamily in the transient receptor potential (TRP) superfamily of ion channels. TRPV4 is a Ca2+-permeable, nonselective cation channel that is thought to be involved in the regulation of systemic osmotic pressure. Earley et al. (2005) describe TRPV4 as being involved in vasodilation.

The sequence of TRPV4 can be found, for example, in the Entrez Nucleotide database maintained by the National Center for Biotechnology Information (NCBI) under the database accession number NM_021625. The feature table of this entry provides the amino acid sequence encoded by the nucleic acid sequence presented in this database entry. The sequence of SEQ ID NO: 2 is identical with the amino acid sequence provided in database entry NM_021625 at the filing date of this patent application. While it is assumed that the sequence provided in database entry NM_021625 is correct, it is noted that any corrected version of this sequence, in case said sequence turned out to be incorrect, is deliberately envisaged for the purposes of the present invention.

In accordance with the present invention the term "polynucleotide" defines a nucleic acid molecule consisting of more than 30 nucleotides. The group of molecules designated as "polynucleotides" also comprises complete genes. The term "polynucleotide" is understood to comprise polydeoxyribonucleotides as well as polyribonucleotides. In other words, all forms of DNA, e.g. genomic DNA and cDNA, and RNA, e.g. mRNA, tRNA, rRNA and genomic RNA (such as in case of RNA viruses) are embraced.

The polynucleotides of (b) include polynucleotides encoding polypeptides comprising or consisting of fragments of the amino acid sequence set forth in SEQ ID NO: 2. It is well known in the art that functional polypeptides may be cleaved to yield fragments with unaltered or substantially unaltered function. Such cleavage may include the removal of a given number of N- and/or C-terminal amino acids. Additionally or alternatively, a number of internal (non-terminal) amino acids may be removed, provided the obtained polypeptide has arteriogenic activity. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged. In particular, removals of amino acids which preserve sequence and boundaries of any conserved functional domain(s) or subsequences in the sequence of SEQ ID NO: 2 are particularly envisaged. Means and methods for determining such domains are well known in the art and include experimental and bioinformatic means. Experimental means include the systematic generation of deletion mutants and their assessment in assays for arteriogenic activity known in the art and as described in the Examples enclosed herewith (see above and Example 3 enclosed herewith). Bioinformatic means include database searches. Suitable databases included protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam; see also Finn et al., Nucleic Acids Res. 34: D247 - D251 (2006)).

The polynucleotides of (c) include polynucleotides which hybridize with the polynucleotides of (a) or (b). It is well known in the art how to perform hybridization experiments with nucleic acid molecules and polynucleotides, i.e. the person skilled in the art knows what hybridization conditions s/he has to use in accordance with the present invention. Such hybridization conditions are referred to in standard text books such as "Molecular Cloning A Laboratory Manual", Cold Spring Harbor Laboratory (1989) N.Y. or Higgins, S.J., Hames, D. "RNA Processing: A practical approach", Oxford University Press (1994), Vol. 1 and 2. "Stringent hybridization conditions" refers to conditions which comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1 x SSC at about 65°C. Said conditions for hybridization are also known by a person skilled in the art as "high stringent conditions for hybridization". Also contemplated are polynucleotides according to (c) that hybridize to the polynucleotides of (a) or (b) at lower stringency hybridization conditions ("low stringent conditions for hybridization"). Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 50°C in 4x SSC or an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

The polynucleotides according to (c) furthermore comprise polynucleotides comprising or consisting of a nucleotide sequence encoding a polypeptide, wherein said polypeptide exhibits at least 75%, 80%, 85%, 90%, 95%, 96%, 97%, 98% or 99% sequence identity with the sequence of the polypeptide encoded by the polynucleotide of (a) or (b) over the entire length, and wherein said polypeptide has arteriogenic activity.

Two nucleotide or protein sequences can be aligned electronically using suitable computer programs known in the art. Such programs comprise BLAST (Altschul et al. (1990), J. Mol. Biol. 215, 403-410), variants thereof such as WU-BLAST (Altschul & Gish (1996), Methods Enzymol. 266, 460-480), FASTA (Pearson & Lipman (1988), Proc. Natl. Acad. Sci. USA 85, 2444-2448) or implementations of the Smith-Waterman algorithm (SSEARCH, Smith & Waterman (1981), J. Mol. Biol. 147, 195-197). These programs, in addition to providing a pairwise sequence alignment, also report the sequence identity level (usually in percent identity) and the probability for the occurrence of the alignment by chance (P-value). Programs such as CLUSTALW (Higgins et al. (1994), Nucleic Acids Res. 22, 4673-4680) can be used to align more than two sequences.

Said polynucleotides include polynucleotides encoding fusion proteins. Consequently, said polypeptides include polypeptides which are fusion proteins. Those components of said fusion proteins, which are not TRPV4 sequences or fragments or variants thereof, as defined herein above, include amino acid sequence which confer desired properties such as modified/enhanced stability, modified/enhanced solubility and/or the ability of targeting one or more specific cell types. For example, fusion proteins with antibodies specific for cell surface markers or with antigen-recognizing fragments of said antibodies are envisaged.

Polypeptides comprised in the pharmaceutical composition of the invention include polypeptides wherein one or more amino acids are chemically modified or replaced with non-naturally occurring amino acid whilst maintaining arteriogenic activity. The term "naturally occurring amino acid" refers to the following amino acids: Ala, Cys, Asp, Glu, Phe, Gly, His, Ile , Lys, Leu, Met, Asn, Pro, Gln, Arg, Ser, Thr, Val, Trp and Tyr. Preferably, up to 20, up to 10, or up to 5 amino acids are chemically modified or replaced with a non-naturally occurring amino acid. Examples of chemically modified amino acids and non-naturally occurring amino acids according to the invention include selenocysteine, β-alanine, α-aminobutyric acid, γ-aminobutyric acid, α-aminoisobutyric acid, norvaline, norleucine, ε-lysine, ornithine, homoserine and hydroxyproline.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of a pharmaceutical composition administered to an individual will, inter alia, depend on the nature of the compound. For example, if said compound is a polypeptide or protein, the total pharmaceutically effective amount of pharmaceutical composition administered parenterally per dose will be in the range of about 1 µg protein /kg/day to 10 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semipermeable polymer matrices in the form of shaped articles, e.g., films, or microcapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The present inventors surprisingly discovered that TRPV4 functions as a positive regulator of arteriogenesis (see Examples enclosed herewith). This is unexpected, noting that the prior art reviewed herein above reports an involvement of TRPV4 in vasodilation mechanisms, i.e. a rapid change in the width of a blood vessel in response to signalling molecules as opposed to arteriogenesis, which is subject of the present invention. "Arteriogenesis" is defined above and involves cell division, increase in tissue mass and occurs over an extended period of time.

The present invention furthermore relates to the use of (a) a polynucleotide comprising or consisting of a nucleotide sequence encoding a polypeptide with the amino acid sequence as set forth in SEQ ID NO: 2; (b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity; (c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or (d) a polypeptide encoded by the polynucleotide of any one of (a) to (c) for the preparation of a pharmaceutical composition for the treatment or prevention of an ischemic condition and/or for the treatment of a patient undergoing tissue engineering.

The term "ischemic" means "affected by ischemia", wherein "ischemia" designates a low oxygen state, for example due to obstruction of the arterial blood supply or inadequate blood flow leading to hypoxia in the tissue. "Ischemia" is a term well known to those skilled in the art and is commonly used to define the condition of a patient. Preferred ischemic conditions according to the invention are detailed further below.

The term "tissue engineering" designates the development of biological substitutes that restore, maintain or improve tissue function. The aims of tissue engineering include the production of functional replacement tissue for clinical use. Tissue engineering may take place in vivo, i.e., tissue is engineered or grown in a patient. It is known (L'heureux et al. (2006)) that during the course of tissue engineering frequently a shortage of blood supply arises. As a consequence, patients undergoing tissue engineering or receiving engineered tissue are in need of a stimulation of arteriogenesis in the region(s) to be engineered. Preferred tissue to be engineered include blood vessels including arteries. Alternatively, and as detailed further below, tissue engineering may take place in vitro or ex vivo.

In a preferred embodiment, said treatment or prevention is effected by the stimulation of arteriogenesis.

In a further preferred embodiment, said pharmaceutical composition is to be delivered to the organ or body part affected by said ischemic condition or undergoing tissue engineering. Delivery to said organ or body part may be effected by local administration to or near the site affected by said ischemic condition or undergoing tissue engineering. Other means of targeted delivery include pharmaceutical compositions which, upon non-local administration accumulate and/or develop efficacy predominantly or exclusively in said organ or body part. For example, antibodies specific for a tissue marker may be employed as means of targeted delivery of a pharmaceutical composition comprising such antibodies in addition to the pharmaceutically active agents according to the invention.

In a preferred embodiment of the pharmaceutical composition or the use according to the invention, said polypeptide exhibits at least 80% or 85% sequence identity. In a further preferred embodiment of the pharmaceutical composition or the use according to the invention, said polypeptide exhibits at least 90%, 95%, 96%, 97%, 98% or 99% sequence identity.

In a further preferred embodiment of the pharmaceutical composition or the use according to the invention, said nucleotide sequence comprises of consists of the sequence set forth in SEQ ID NO: 1.

The sequence of SEQ ID NO: 1 is the sequence of the mRNA for the human TRPV4 protein. The sequence can be found, for example, in the Entrez Nucleotide database maintained by the National Center for Biotechnology Information (NCBI) under the database accession number NM_021625. The sequence of SEQ ID NO: 1 is identical with the sequence provided in database entry NM_021625 at the filing date of this patent application. While it is assumed that the sequence provided in database entry NM_021625 is correct, it is noted that any corrected version of this sequence, in case said sequence turned out to be incorrect, is deliberately envisaged for the purposes of the present invention.

Also included within the scope of the invention are polynucleotides which differ from the sequence set forth in SEQ ID NO: 1 as a consequence of the degeneracy of the genetic code, i.e., the envisaged polynucleotides, while different in sequence from the sequence set forth in SEQ ID NO: 1, encode the same polypeptide as does the sequence set forth in SEQ ID NO: 1.

In a further preferred embodiment of the pharmaceutical composition or the use according to the invention, said nucleotide sequence is comprised in a vector.

The vector of the present invention may be, e.g., a plasmid, cosmid, virus, bacteriophage or another vector used e.g. conventionally in genetic engineering, and may comprise further genes such as marker genes which allow for the selection of said vector in a suitable host cell and under suitable conditions.

Furthermore, the vector of the present invention may, in addition to the nucleotide sequences of the invention, comprise expression control elements, allowing proper expression of the coding regions in suitable hosts. Such control elements are known to the artisan and may include a promoter, a splice cassette, translation initiation codon, translation and insertion site for introducing an insert into the vector. Preferably, the nucleotide sequence of the invention is operably linked to said expression control sequences allowing expression in eukaryotic or prokaryotic cells.

Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook (1989), loc. Cit., and Ausubel, Current Protocols in Molecular Biology, Green Publishing Associates and Wiley Interscience, N.Y. (1989), (1994). Alternatively, the nucleotide sequences and vectors of the invention can be reconstituted into liposomes for delivery to target cells. Thus, according to the invention relevant sequences can be transferred into expression vectors where expression of a particular polypeptide/protein is required. Typical cloning vectors include pBscpt sk, pGEM, pUC9, pBR322 and pGBT9. Typical expression vectors include pTRE, pCAL-n-EK, pESP-1, pOP13CAT.

The term "control sequence" or "control element" refers to regulatory DNA sequences which are necessary to effect the expression of coding sequences to which they are ligated. The nature of such control sequences differs depending upon the host organism. In prokaryotes, control sequences generally include promoter, ribosomal binding site, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the vector of the invention is preferably an expression vector. An "expression vector" is a construct that can be used to transform a selected host cell and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotic and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, e.g., the P_{L}, *lac, trp* or *tac* promoter in *E. coli*, and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL). An alternative expression system which could be used is an insect system. In one such system, *Autographa californica* nuclear polyhedrosis virus (AcNPV) is used as a vector to express foreign genes in *Spodoptera frugiperda* cells or in *Trichoplusia* larvae. The coding sequence of a polynucleotide of the invention may be cloned into a nonessential region of the virus, such as the polyhedrin gene, and placed under control of the polyhedrin promoter. Successful insertion of said coding sequence will render the polyhedrin gene inactive and produce recombinant virus lacking coat protein. The recombinant viruses are then used to infect *S. frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227).

In a preferred embodiment, said polynucleotide or said vector is comprised in a liposome. Liposome-mediated transfer of polynucleotides is well known in the art (see above).

In another preferred embodiment, said vector is an adenoviral vector, adeno-associated viral vector or lentiviral vector. Adeno-associated viral vectors and lentiviral vectors are particularly preferred in those cases where the patient recently went through an infection by an adenovirus. In such a case the presence of antibodies directed against said adenovirus may prevent success of a therapy using the adenoviral vector while said antibodies are not expected to recognize antigens presented by said adeno-associated viral vectors.

In a more preferred embodiment, said pharmaceutical composition comprising a vector is to be delivered by means of a catheter to be inserted into the vessel to be treated.

In another more preferred embodiment, said pharmaceutical composition comprising a polypeptide is to be administered by injection at or near the site to be treated.

In a further preferred embodiment, said nucleotide sequence has been transfected into a cell obtained from a patient to be treated. Preferably, said cells are blood cells obtained from a peripheral vein of the patient to be treated. The cells obtained from the patient may be cultured and propagated in vitro prior to transfection. Preferred cells to be obtained and transfected are monocytes of the peripheral blood. Monocytes are amenable to infection by viral vectors and exhibit advantageous homing properties. More specifically, monocytes are capable of attaching to growing collateral vessels (Schaper et al. (1976), WO 00/60054). Upon transfection, the cells are to be re-introduced into the patient. Re-introducing can be done by injection into or near the collateral vessel(s). Alternatively, simple intravenous injection may be performed. In this case, a co-transfection with Mac-1 cDNA is preferred. Expression of the Mac-1 gene in the transfected cells ensures that the transfected cells bind to docking sites in the collateral vessel(s).

In a further preferred embodiment of the uses according to the invention, said ischemic condition is a consequence of arterial injury, arterial degeneration, arterial occlusion or arterial re-occlusion.

Preferably, said ischemic condition is selected from atherosclerosis, angina pectoris, infarct, heart attack, stroke, renal infarct, hypertension, wounds, peripheral ischemic vascular disease, bone fracture and abortus imminens. Wounds include wounds which involve arterial lesions. Other ischemic conditions, which are also envisaged, include ischemic conditions arising from diabetes or smoking.

In a further preferred embodiment of the uses according to the invention, the affected organs or body parts are selected from heart, brain, kidneys, limbs and blood vessels. Blood vessels include arteries and veins.

In a further preferred embodiment, said pharmaceutical composition comprises said nucleotide sequence and/or said polypeptide as the sole pharmaceutically active agent(s).

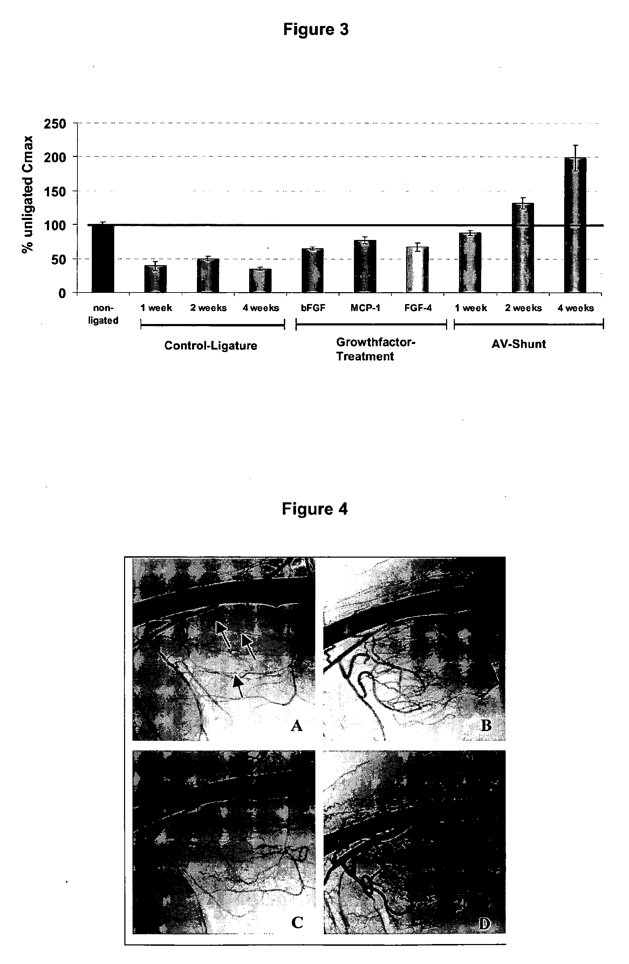

Another preferred embodiment relates to uses and pharmaceutical compositions, wherein said pharmaceutical compositions additionally comprise one or more angiogenic growth factor(s). Angiogenic growth factors have described in the art in their role as stimulators of angiogenesis, which, as described above, is to held distinct from arteriogenesis. As exemplified in Example 3 and Figure 3, the present inventors could show that angiogenic growth factors exhibit a stimulatory effect also on arteriogenesis. While it is expected that angiogenic growth factors fail to elicit an effect comparable to TRPV4 polynucleotides or polypeptides, it is expected that their co-administration with said TRPV4 polynucleotides and/or polypeptides further enhances the stimulatory effect on arteriogenesis. In a preferred embodiment, the combined administration of the nucleotide sequence of the invention and/or the polypeptide of the invention on the one side and of said one or more angiogenic growth factor(s) on the other side exhibits a synergistic effect on arteriogenesis and accordingly in the treatment of the recited diseases.

Preferably, said angiogenic growth factor is selected from the group consisting of FGF-2 (bFGF), FGF-4, MCP-1, PDGF and VEGF.

The present invention furthermore relates to an in vitro or ex vivo method of ensuring blood supply in the course of tissue engineering, wherein the method comprises bringing the tissue into contact with (a) a polynucleotide comprising or consisting of a nucleotide sequence encoding a polypeptide with the amino acid sequence as set forth in SEQ ID NO: 2; (b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity; (c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or (d) a polypeptide encoded by the polynucleotide of any one of (a) to (c). This embodiment relates to the filed of regenerative medicine and pertains to growth and/or modification of tissue outside the human or animal body. As noted above, conditions of limiting blood supply may arise during tissue engineering. As a consequence, stimulation of arteriogenesis in the tissue is desirable. Preferred tissue to be engineered include blood vessels including arteries.

Another subject of the present invention is a method of identifying modulators of the biological activity of the polynucleotide or the polypeptide as defined in the main embodiment, comprising the following steps: (a) bringing into contact said polynucleotide or polypeptide with a test compound; and (b) comparing the biological activity in the presence of said test compound with the biological activity in the absence of said test compound, wherein the biological activity in the presence of a modulator and in the absence thereof differ, wherein said biological activity is the expression of said polynucleotide and/or the stimulation of arteriogenesis. This embodiment relates to a screening method for compounds acting as modulators. Step (a) is effected under conditions which allow the test compound to interact physically and/or functionally with said polynucleotide or polypeptide. Suitable conditions depend on the test compounds. The skilled person is well aware of such suitable conditions which include, for example, buffered solutions comprising the polynucleotide or polypeptide, the test compound(s) and, if required, any further factors necessary for function and integrity of said polynucleotide or polypeptide and/or of said test compound(s).

The term "expression of said polynucleotide" refers to transcription and or translation. Accordingly, means of quantifying expression include determining the amount of mRNA and/or the amount or polypeptide encoded by said polynucleotide.

Methods for the determination of mRNA expression levels are known in the art and comprise Real Time PCR, Northern blotting and hybridization on microarrays or DNA chips equipped with one or more probes or probe sets specific for transcripts encoding polypeptides according to the invention.

The skilled person is aware of methods for the quantitation of polypeptides. Amounts of purified polypeptide in solution can be determined by physical methods, e.g. photometry. Methods of quantifying a particular polypeptide in a mixture rely on specific binding, e.g of antibodies. Specific detection and quantitation methods exploiting the specificity of antibodies comprise immunohistochemistry (*in situ*) and surface plasmon resonance. Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction.

Preferably, said method of identifying modulators is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

Preferably, said modulator is an activator. The term "activator" according to the invention designates a test compound which increases the biological activity of said polynucleotide or polypeptide by 10%, 20%, 30%, 40% or 50%, more preferred by 60%, 70%, 80%, 90% or 100%, and yet more preferred by 200%, 500%, 1000% or more. Activators obtained by the screening method of the invention are suitable for treatment and prevention of ischemic conditions.

In an alternative preferred embodiment, said modulator is an inhibitor. An inhibitor is a compound which decreases the biological activity of said polynucleotide or polypeptide. Preferably, the level of activity is less than 90%, more preferred less than 80%, 70%, 60% or 50% of the activity in absence of the inhibitor. Yet more preferred are inhibitors lowering the level down to less than 25%, less than 10%, less than 5% or less than 1% of the activity in absence of the inhibitor. Inhibitors obtained by the screening method of the invention are suitable for the treatment and prevention of restenosis (see below for further details).

In a preferred embodiment, the method comprises, after step (a) and prior to step (b) the further step of (a') determining whether said test compound binds to said polynucleotide or polypeptide. The determination of binding test compounds in step (a') relates to any binding assay, preferably biophysical binding assay, which may be used to identify binding test molecules prior to performing the functional/activity assay with the binding test molecules only. Suitable biophysical binding assays are known in the art and comprise fluorescence polarization (FP) assay, fluorescence resonance energy transfer (FRET) assay and surface plasmon resonance (SPR) assay. Step (a') is particularly advantageous if said binding assay is more amenable to high throughput than the functional assay. Preferably, only test compounds exhibiting binding affinity to said polynucleotide or polypeptide are fed into step (b) of comparing biological activity in presence and absence of the test compound.

In a further preferred embodiment of the method of identifying modulators, said polynucleotide and/or said polypeptide is/are comprised in a cell transfected with said polynucleotide. This embodiment relates to a cellular screen. In a cellular screen modulators may be identified which exert their modulatory activity by physically interacting with the target molecule (said polynucleotide or said polypeptide), or alternatively (or additionally) by functionally interacting with said target molecule, i.e., by interfering with the pathway(s) present in the cells employed in the cellular assay and in control of the biological activity of said polynucleotide or said polypeptide.

In another preferred embodiment of the method of identifying modulators of the invention, the method comprises the further step of (c) formulating one or more modulators obtained with a pharmaceutically acceptable carrier, excipient or diluent.

In a more preferred embodiment, the method of identifying modulators comprising, after step (b) and prior to step (c) the step of (b') optimizing the pharmacological properties of the modulator.

Methods for the optimization of the pharmacological properties of compounds identified in screens, generally referred to as lead compounds, are known in the art and comprise a method of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The present invention also relates to an antibody identified or obtainable by said method of identifying modulators. Preferably said antibody is an antibody capable of binding the polypeptide defined in the main embodiment. More preferably, said binding is specific. Methods for raising antibodies against a given polypeptide or protein are well know in the art.

Whether an antibody according to the invention acts as an activator or inhibitor of the biological activity of the polypeptides of the invention depends on the region of the polypeptide to which said antibody binds. Depending on said region different states such as conformational states of said polypeptide may be stabilized. If a state of said polypeptide is stabilized which exhibits greater arteriogenic activity, the corresponding antibody is an activator according to the invention. On the other hand, if a state of said polypeptide is stabilized which exhibits lower arteriogenic activity, the corresponding antibody is an inhibitor according to the invention. Means and methods of quantifying arteriogenic activity are described herein. Accordingly, determining whether an antibody of the invention is an inhibitor or activator can be performed without further ado.

The term "antibody" includes monoclonal antibodies, polyclonal antibodies, single chain antibodies, or fragments thereof that specifically bind said polypeptide, also including bispecific antibodies, synthetic antibodies, antibody fragments, such as Fab, a F(ab₂)', Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the aforementioned polypeptides can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of the peptide or polypeptide of the invention (Schier, Human Antibodies Hybridomas 7 (1996), 97-105; Malmborg, J. Immunol. Methods 183 (1995), 7-13). The production of chimeric antibodies is described, for example, in WO89/09622. A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook (1989), loc. cit..

The term "monoclonal" or "polyclonal antibody" (see Harlow and Lane, (1988), loc. cit.) also relates to derivatives of said antibodies which retain or essentially retain their binding specificity. Preferred derivatives of such antibodies are chimeric antibodies comprising, for example, a mouse or rat variable region and a human constant region.

The term "scFv fragment" (single-chain Fv fragment) is well understood in the art and preferred due to its small size and the possibility to recombinantly produce such fragments.

The term "specifically binds" in connection with the antibody used in accordance with the present invention means that the antibody etc. does not or essentially does not cross-react with polypeptides of similar structures. Cross-reactivity of a panel of antibodies etc. under investigation may be tested, for example, by assessing binding of said panel of antibodies etc. under conventional conditions (see, e.g., Harlow and Lane, (1988), loc. cit.) to the polypeptide of interest as well as to a number of more or less (structurally and/or functionally) closely related polypeptides. Only those antibodies that bind to the polypeptide/protein of interest but do not or do not essentially bind to any of the other polypeptides which are preferably expressed by the same tissue as the polypeptide of interest, are considered specific for the polypeptide/protein of interest.

In a particularly preferred embodiment of the method of the invention, said antibody or antibody binding portion is or is derived from a human antibody or a humanized antibody.

The term "humanized antibody" means, in accordance with the present invention, an antibody of non-human origin, where at least one complementarity determining region (CDR) in the variable regions such as the CDR3 and preferably all 6 CDRs have been replaced by CDRs of an antibody of human origin having a desired specificity. Optionally, the non-human constant region(s) of the antibody has/have been replaced by (a) constant region(s) of a human antibody. Methods for the production of humanized antibodies are described in, e.g., EP-A1 0 239 400 and WO90/07861.

Antibodies according to the invention which act as modulators of biological activity can be obtained or identified by subjecting antibodies to the method of identifying modulators according to the invention.

The present invention furthermore provides the use of an activator of a polypeptide, said polypeptide being defined in the main embodiment, for the preparation of a pharmaceutical composition for the treatment or prevention of an ischemic condition and/or for the treatment of a patient undergoing tissue engineering. Also provided is an activator of a polypeptide, said polypeptide being defined in the main embodiment, for the treatment or prevention of an ischemic condition and/or for the treatment of a patient undergoing tissue engineering.

The term "activator" refers to compounds which increase the arteriogenic activity of said polypeptide as defined in the main embodiment. Means and methods for determining and quantifying arteriogenic activity are described herein.

In a preferred embodiment, said activator is a cation channel opener. Preferably, said cation channel opener is an opener of the cation channel encoded by the polynucleotide defined in the main embodiment. More preferred, said cation channel opener is a TRPV4 channel opener. Cation channel openers can readily be determined by determining the Ca²⁺ influx across the cell membrane of cells expression a cation channel. For example, HEK cells overexpressing the polypeptide as defined in the main embodiment may be used for that purpose. HEK cells overexpressing TRPV4 as well as means and methods for determining Ca²⁺ influx are described in, for example, Earley et al. (2005).

In a further preferred embodiment, said activator is an epoxy-eicosatrienoic acid, preferably an 11,12-epoxy-eicosatrienoic acid, more preferred 11,12-epoxy-eicosa-5,8,14-trienoic acid. Epoxy-eicosatrienoic acids include cytochrome P450 epoxygenase products of arachidonic acid (5,8,11,14-eicosatetraenoic acid).

The present invention also relates to the use of an inhibitor of a polypeptide, said polypeptide being defined in the main embodiment, for the preparation of a pharmaceutical composition for the treatment or prevention of restenosis following stent implementation. Also provided is an inhibitor of a polypeptide, said polypeptide being defined in the main embodiment, for the treatment or prevention of restenosis following stent implementation.

The term "inhibitor" refers to compounds which decrease the arteriogenic activity of said polypeptide as defined in the main embodiment.

The term "stent" is well known in the art and includes expandable wire meshes and hollow perforated tubes that are inserted into a hollow structure of the body to keep it open. Said hollow structures to be kept open include blood vessels which in turn include arteries. It is well known in the art that upon implementation of a stent, e.g. by insertion into an artery by surgical means, the inner width of said artery has a tendency to decrease over time which may eventually lead to re-occlusion. Such a condition is referred to as restenosis.

In a preferred embodiment of the use of the invention, said pharmaceutical composition comprises a stent. The pharmaceutical composition of the invention comprising a stent may be a stent coated with said inhibitor. Any constituent of a pharmaceutical composition of the invention (besides the stent itself) may be comprised in a coating layer of a stent according to the invention.

Also provided is a pharmaceutical composition comprising or consisting of a stent, an inhibitor of a polypeptide, the polypeptide being defined in the main embodiment, and optionally a pharmaceutically acceptable carrier.

Preferably, said inhibitor is selected from the group consisting of (a) an siRNA, an antisense nucleic acid, a ribozyme binding specifically the polynucleotide defined in the main embodiment; (b) an antibody, aptamer, and a fragment or derivative thereof binding specifically the polypeptide defined in the main embodiment.

The term "small interfering RNA" (siRNA), sometimes known as short interfering RNA or silencing RNA, refers to a class of generally short and double-stranded RNA molecules that play a variety of roles in biology and, to an increasing extent, in treatment of a variety of diseases and conditions. Most notably, siRNA is involved in the RNA interference (RNAi) pathway where the siRNA interferes with the expression of a specific gene (see, e.g. Zamore Nat Struct Biol 2001, 8(9):746-50; Tuschl T. CHEMBIOCHEM. 2001, 2:239-245; Scherr and Eder, Cell Cycle. 2007 Feb;6(4):444-9; Leung and Whittaker, Pharmacol Ther. 2005 Aug;107(2):222-39; de Fougerolles et al., Nat. Rev. Drug Discov. 2007, 6: 443-453).

Such siRNAs are generally 18-27 nt long, generally comprising a short (usually 19-21-nt) double-strand of RNA (dsRNA) with or without 2-nt 3' overhangs on either end. Each strand can have a 5' phosphate group and a 3' hydroxyl (-OH) group or the phosphate group can be absent on one or both strands. This structure is the result of processing by dicer, an enzyme that converts either long dsRNAs or small hairpin RNAs into siRNAs.

siRNAs can also be exogenously (artificially) introduced into cells by various transfection methods to bring about the specific knockdown of a gene of interest. In this context, other structures than those described above are also envisaged, provided they are capable of interfering with gene expression. Preferably, the double-stranded part has a length of about 12 to about 50 base pairs in length. The siRNA of the invention may either have overhanging sequences of up to 10 bases, preferably not more than 5 bases in length at either 3' end or at one 3' end, or may be bluntended. Also preferred is that the complementarity to the target gene extends over the entire length of the double-stranded part. The region which is complementary to the target gene is at least 12 bases, preferably at least 15, 16, 17, 18, 19, 20, 21, 22, 23 or more bases in length. The siRNA of the invention may be fully complementary to the target gene. Alternatively, the siRNA may comprise up to 5%, 10%, 20% or 30% mismatches to the target gene. Furthermore, siRNAs and also antisense RNAs can be chemically modified e.g. on the backbone including the sugar residues. Preferred modifications of the siRNA molecules of he invention include linkers connecting the two strands of the siRNA molecule. Chemical modifications serve inter alia to improve the pharmacological properties of siRNAs and antisense RNAs such as in vivo stability and/or delivery to the target site within an organism. The skilled person is aware of such modified siRNAs as well as of means and methods of obtaining them, see, for example, Zhang et al., Curr Top Med Chem. 2006;6(9):893-900; Manoharan, Curr Opin Chem Biol. 2004 Dec;8(6):570-9.

Essentially any gene of which the sequence is known can thus be targeted based on sequence complementarity with an appropriately tailored siRNA. This has made siRNAs an important tool for gene function and drug target validation studies as well as for therapeutic intervention which is envisaged here. The siRNAs of the present invention are capable of reducing or blocking expression of the proteins and nucleic acids defined herein above.

The term "antisense nucleic acid" is known in the art and refers to a nucleic acid which is complementary to a target nucleic acid, here to a nucleic acid according to the main embodiment. An antisense molecule according to the invention is capable of interacting with, more specifically hybridizing with said nucleic acid. By formation of the hybrid, expression of the target mRNA is reduced or blocked. Standard methods relating to antisense technology have been described (see, e.g., Melani et al., Cancer Res. (1991) 51:2897-2901; Aboul-Fadl, Curr Med Chem. 2005; 12:2193-214; Pirollo et al., Pharmacol Ther. 2003; 99:55-77).

Ribozymes are enzymes consisting of or comprising a catalytically active ribonucleic acid which in turn comprises a sequence complementary to a sequence in the target nucleic acid. Ribozymes specifically cleave the target nucleic acid, such as the (pre)-mRNA of a gene, the consequence being reduction or repression of expression. The techniques underlying said repression of expression are well known in the art (see, e.g., EP-B1 0 291 533, EP-A1 0 321 201, EP-A2 0 360 257). Selection of appropriate target sites and corresponding ribozymes can be done as described for example in Steinecke et al. (Methods in Cell Biology (1995) 50:449-460). Ribozymes according to the invention are capable of cleaving nucleic acids defined in the main embodiment.

The term "aptamer" as used herein refers to DNA or RNA molecules that have been selected from random pools based on their ability to bind other molecules. Aptamers have been selected which bind nucleic acid, proteins, small organic compounds, and even entire organisms. A database of aptamers is maintained at http://aptamer.icmb.utexas.edu/; see also Lee et al., Nucleic Acids Research, 2004, Vol. 32, Database issue D95-D100.

The Figures show:

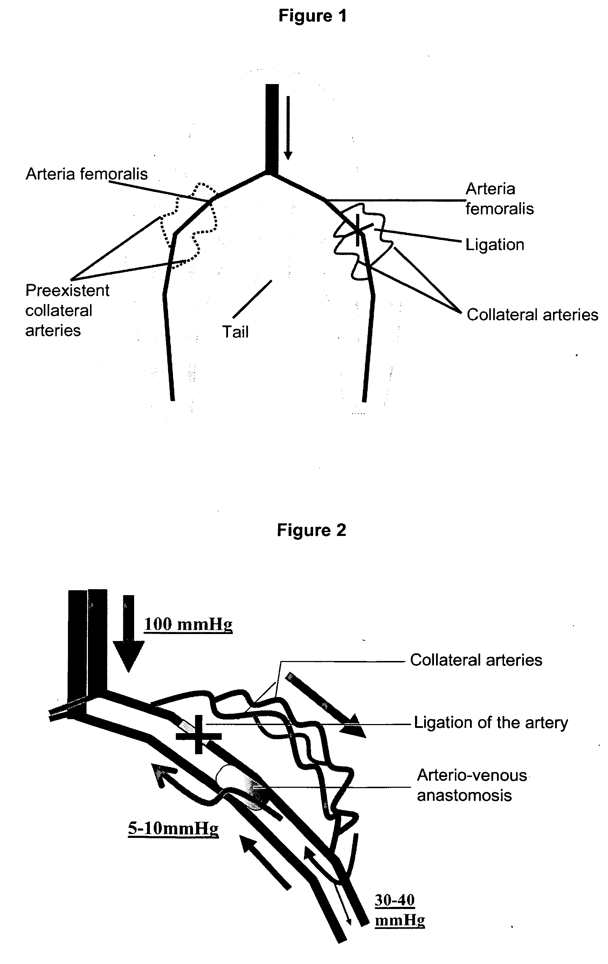

**Figure 1****:** Schematic illustration of the model of femoral artery ligation in the hindlimb of a rabbit. In the left limb the unoccluded arteria with pre-existent collateral arteries is shown. In the right limb the artery is occluded (ligation) and the collateral arteries enlarged to assure perfusion of the distal limb (arteriogenesis).
**Figure 2****:** Shown is a magnified part of the vascular system of one limb. The artery is ligated (Ligation) and the collateral arteries bypass this occlusion.
   Below the ligation an arterio-venous anastomosis is prepared, because of the low pressure in the vein (5-10mmHg), in contrast to the higher pressure in the lower limb (30-40mmHg) the main fraction of the blood flows out of the collaterals directly into the vein.
**Figure 3****:** Results of the measurements of maximal collateral conductances in % of Ccmax of unoccluded femoral arteries in contrast to growth factor treated and arterio-venous-(AV-)shunt treated collateral systems. The aim to reach conductances of non-ligated femoral arteries was not reached in control-ligatures and after growth factor treatments. After one week shunt treatment this aim is reached, after two weeks it is surpassed and after 4 weeks the conductance is doubled in contrast to unligated arteries.
**Figure 4****:** *Post mortem* Angiographies of collateral systems after different treatments A) Ligature of the artery without any further treatment after 1 week: only small collaterals are visible (arrows) B) Ligature plus shunt treatment after 1 week: a lot of collaterals with a larger diameter are visible C) Ligature without any further treatment after 4 weeks: some collaterals are visible D) Ligature plus shunt treatment after 4 weeks: huge amount of large collateral arteries.

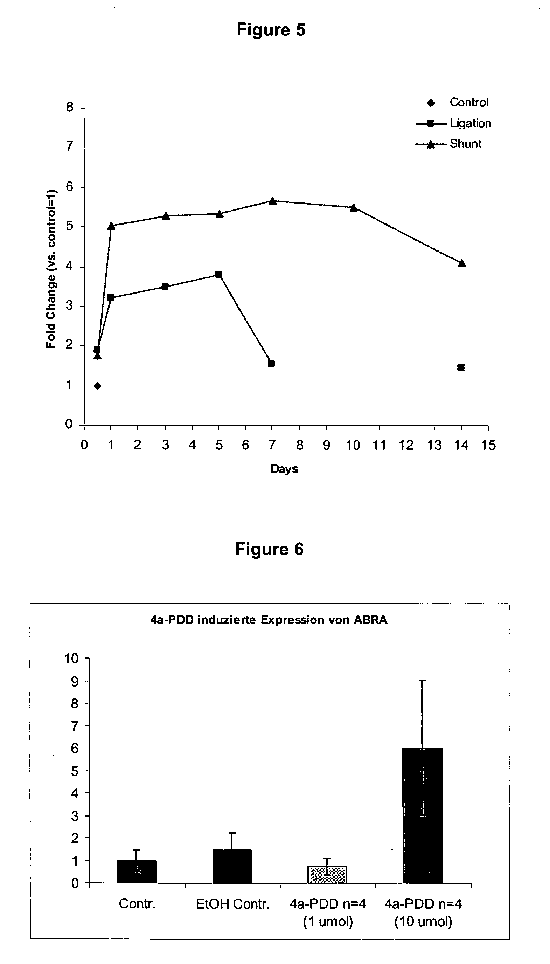

**Figure 5****:** Time course of TRPV4 transcription in AV-shunt vs. control and ligation of the femoral artery in rats.
**Figure 6****:** ABRA mRNA-expression of rat smooth muscle cells after treatment with 4α-PDD, a TRPV4 channel opener. When using a concentration of 10 µmol ABRA transcription was significantly increased.

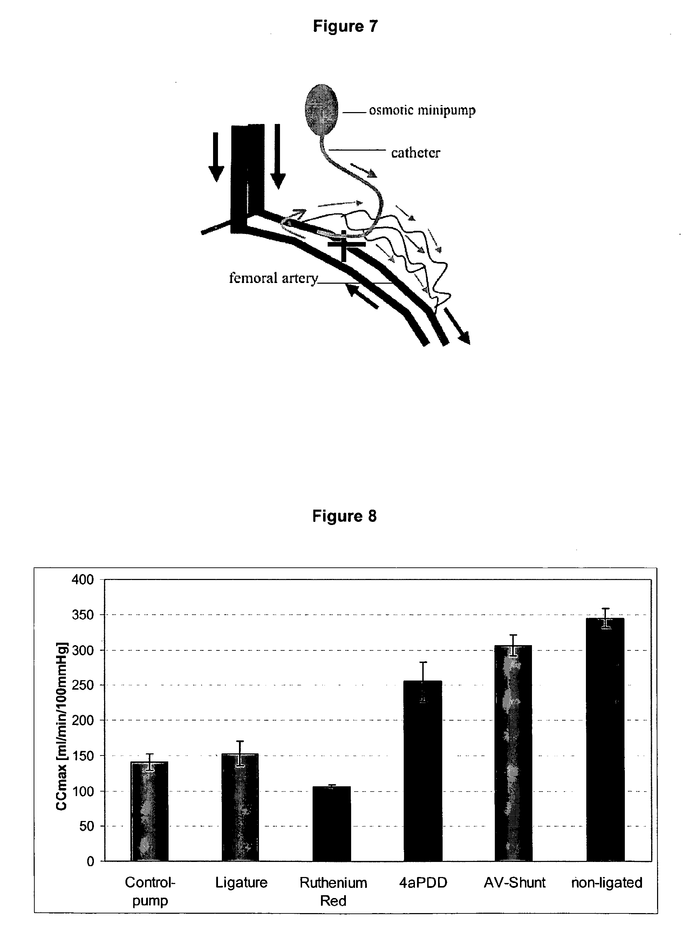

**Figure 7****:** The osmotic minipump was filled with the solvent of interest. The catheter which was connected to the osmotic minipump was placed proximally to the ligation in the femoral artery. After implantation the substance was delivered continuously and during first-pass effect into the collateral circulation.
**Figure 8****:** The stimulatory effects of 4αPDD and inhibitory effect of Ruthenium Red via osmotic minipumps are shown. The blue bars show the respective control groups for 4aPDD- , Ruthenium Red- or AV-shunt-treatment. Also shown is the maximum conductance of non ligated femoral arteries (purple).

The following examples illustrate the invention but should not be construed as being limiting.

### Example 1

### The animal model

To induce arteriogenesis in rabbits, the femoral artery is ligated, which is the main artery of the hindlimb (Fig. 1). Then a direct connection between the artery distal to the ligation and the accompanying vein is established (Fig. 2).

### Example 2

### Maximum collateral conductance measurements

Hemodynamic measurements were performed to quantify the maximum capacity of the collateral system on the basis of measuring the *maximum collateral conductance (Ccmax)*. Methods of measuring the maximum collateral conductance are well known to the person skilled in the art and can be performed as outlined in the description and as described, for example, in Schaper et al. (1976) und Pasyk et al. (1982).

In particular, for the terminal experiment the animals were anesthetized again and both iliac arteries were exposed and outfitted with ultrasonic flow probes to measure blood flow in ml/min. These were positioned proximal to the deep femoral artery, i.e., the stem of most of the growing collateral vessels. A small PE 50 catheter was advanced through a side-branch into the abdominal aorta for the infusion of adenosine. A fluid filled catheter was introduced into the carotid artery for the measurement of systemic arterial pressure in mmHg. Both pedal arteries were prepared and catheters inserted for the measurement of the pressure gradient over the collateral network or along the intact femoral artery and its tributaries. Adenosine was infused at increasing concentrations until the central aortic pressure began to fall. This happened usually at a concentration of 600 micrograms/kg per minute. Pressure and flow signals were fed into an AD-converter and were digitally recorded on a MacLab computer. Maximal conductances (Cmax) were calculated by dividing maximal adenosine-recruited flows by the pressure gradient across the collateral network (ml/min/mmHg).

The aim was to reach maximum conductances comparable to those of unligated femoral arteries, that means to reach a complete restoration of arterial function. Prior to establishing the shunt experiments, even after treatment with different growth factors, it was not possible to reach a full recovery. To reach a full recovery (Fig. 3) means that "super-bypasses" have been created. These "super-bypasses" are visible in *post mortal* angiographies (Fig. 4).

### Example 3

### Differential expression

To perform molecular biology experiments, the shunt model was also established in the rat. The rat angiographies showed, that shunt treatment stimulates arteriogenesis in rats to the same degree as in rabbits.

From dissected rat collateral arteries total RNA was isolated and subjected to micro array analysis (Rat whole Genome Array, Agilent).

Among the significantly up-regulated transcripts surprisingly *TRPV4* (transient receptor potential cation channel, subfamily V, member 4) was identified. Using quantitative PCR an up to 5.5-fold increase of *TRPV4* transcripts was confirmed 7 days after shunt surgery.

To investigate the time course of TRVP4 transcription, total RNA from collateral arteries was isolated at different time points (1d, 3d, 5d, 7d, 10d and 14d) after shunt implementation and subjected to qRT PCR analysis (Fig. 5). Whereas in control collaterals (ligation without shunt) the transcription peaks at day 5 and drops after seven days, in "shunt" collaterals *TRPV4* is transcribed at much higher levels and still significantly upregulated after 14 days.

### Example 4

### Effect of TRPV4 openers

In cultivated rat SMC (A10) that are treated with 4α-phorbol-12,13-didecanoate (4α-PDD) (10 uM/l), a selective *TRPV4*-opener, we could show that transcription of actin binding Rho-activator (ABRA) increases (Fig. 6). *ABRA* is a downstream mediator of collateral growth which leads to activation of the Rho/ROCK-signaling pathway. This Pathway is crucially involved in the remodelling processes of collateral arteries after an arterial occlusion.

In further experiments, the direct effect of 4αPDD on arteriogenesis was assessed. 4αPDD was locally infused at a concentration of 2.4 µg/kg body weight/day over 7 days. The hemodynamic results revealed a strong stimulatory effect of 4αPDD (255±28,27 ml/min/100mmHG) vs. control (152,8±17,0 ml/min/100mmHG)

### Example 5

### Effect of TRPV4 inhibitors

In order to test the consequences of a functionally altered *TRPV4* channel inside the collateral system of rabbits, after femoral artery ligation a osmotic mini pump was used to administer Ruthenium Red, a *TRPV4*-channel blocker, or 4α-PDD, a *TRPV4-*channel opener. The femoral artery was cannulated with a sterile catheter with the tip positioned approximately 1 cm distal to the branches of the feeding arteries of the collateral system. The catheter was connected to the osmotic munipump which was subcutaneously fixed in the flank. This method ensures a chronically and local release of the substance directly into the collateral circulation (Fig. 7) and is described in detail in Hoefer et al., Cardiovasc. Res. 2001;49:609-17.

By using Ruthenium Red, in the hind limb ischemic model in rabbits over a time period of 2 weeks, we could demonstrate that, after ligation of the femoral artery, collateral growth is inhibited by 67 % compared to the ligation control. CCmax of the collateral system is markedly decreased (106 ml/min/mmHg) in ruthenium red treated side compared to the intra individual control side, harbouring just the ligation of the femoral artery (162 ml/min/mmHg) (Fig. 8).

### Further References

Earley et al. (2005). Circ. Res. 97, 1270-1279.
L'heureux et al. (2006). Nat. Med. Feb. 19 (electronic publication ahead of print).
Pasyk et al. (1982). Am. J. Physiol. 242, H1031-1037.
Schaper, J. et al. (1972). Am. J. Cardiol. 29, 851-859.
Schaper, J. et al., (1976). Virchows Arch. A. Pathol. Anat. Histol. 370, 193-205.
Schaper, W. et al. (1969). Cardiovasc. Res. 3, 315-323.
Schaper, W. (1971). The Collateral Circulation of the Heart. New York: American Elsevier Publishing Company. ISBN 0-7204-7300-4.
Schaper, W. et al. (1971). Circ. Res. 28, 671-679.

aaaaaaaaaa aaaa

<210> 2
<211> 871
<212> PRT
<213> homo sapiens

<400> 2

Met Ala Asp Ser Ser Glu Gly Pro Arg Ala Gly Pro Gly Glu Val Ala
1               5                   10                  15

Glu Leu Pro Gly Asp Glu Ser Gly Thr Pro Gly Gly Glu Ala Phe Pro
            20                  25                  30

Leu Ser Ser Leu Ala Asn Leu Phe Glu Gly Glu Asp Gly Ser Leu Ser
        35                  40                  45

Pro Ser Pro Ala Asp Ala Ser Arg Pro Ala Gly Pro Gly Asp Gly Arg
    50                  55                  60

Pro Asn Leu Arg Met Lys Phe Gln Gly Ala Phe Arg Lys Gly Val Pro
65                  70                  75                  80

Asn Pro Ile Asp Leu Leu Glu Ser Thr Leu Tyr Glu Ser Ser Val Val
                85                  90                  95

Pro Gly Pro Lys Lys Ala Pro Met Asp Ser Leu Phe Asp Tyr Gly Thr
            100                 105                 110

Tyr Arg His His Ser Ser Asp Asn Lys Arg Trp Arg Lys Lys Ile Ile
        115                 120                 125

Glu Lys Gln Pro Gln Ser Pro Lys Ala Pro Ala Pro Gln Pro Pro Pro
    130                 135                 140

Ile Leu Lys Val Phe Asn Arg Pro Ile Leu Phe Asp Ile Val Ser Arg
145                 150                 155                 160

Gly Ser Thr Ala Asp Leu Asp Gly Leu Leu Pro Phe Leu Leu Thr His
                165                 170                 175

Lys Lys Arg Leu Thr Asp Glu Glu Phe Arg Glu Pro Ser Thr Gly Lys
            180                 185                 190

Thr Cys Leu Pro Lys Ala Leu Leu Asn Leu Ser Asn Gly Arg Asn Asp
        195                 200                 205

Thr Ile Pro Val Leu Leu Asp Ile Ala Glu Arg Thr Gly Asn Met Arg
    210                 215                 220

3

SEQUENCE LISTING

<110>  Max-Planck-Gesellschaft zur Förderung der Wissenschaften
       e.V.

<120>  Means and methods for treating peripheral and cardiovascular
       diseases via modulation of arteriogenesis

<130>  N1102 EP

<160>  2

<170>  PatentIn version 3.3

<210>  1
<211>  3254
<212>  DNA
<213>  homo sapiens

<400>  1
ccggccggga ttcaggaagc gcggatctcc cggccgccgg cgcccagccg tcccggaggc      60

tgagcagtgc agacgggcct ggggcaggca tggcggattc cagcgaaggc ccccgcgcgg     120

ggcccgggga ggtggctgag ctccccgggg atgagagtgg caccccaggt ggggaggctt     180

ttcctctctc ctccctggcc aatctgtttg agggggagga tggctccctt tcgccctcac     240

cggctgatgc cagtcgccct gctggcccag gcgatgggcg accaaatctg cgcatgaagt     300

tccagggcgc cttccgcaag ggggtgccca accccatcga tctgctggag tccaccctat     360

atgagtcctc ggtggtgcct gggcccaaga aagcacccat ggactcactg tttgactacg     420

gcacctatcg tcaccactcc agtgacaaca agaggtggag gaagaagatc atagagaagc     480

agccgcagag ccccaaagcc cctgcccctc agccgccccc catcctcaaa gtcttcaacc     540

ggcctatcct ctttgacatc gtgtcccggg gctccactgc tgacctggac gggctgctcc     600

cattcttgct gacccacaag aaacgcctaa ctgatgagga gtttcgagag ccatctacgg     660

ggaagacctg cctgcccaag gccttgctga acctgagcaa tggccgcaac gacaccatcc     720

ctgtgctgct ggacatcgcg gagcgcaccg gcaacatgcg ggagttcatt aactcgccct     780

tccgtgacat ctactatcga ggtcagacag ccctgcacat cgccattgag cgtcgctgca     840

aacactacgt ggaacttctc gtggcccagg gagctgatgt ccacgcccag gcccgtgggc     900

gcttcttcca gcccaaggat gaggggggct acttctactt tggggagctg cccctgtcgc     960

tggctgcctg caccaaccag ccccacattg tcaactacct gacggagaac ccccacaaga    1020

aggcggacat gcggcgccag gactcgcgag gcaacacagt gctgcatgcg ctggtggcca    1080

ttgctgacaa cacccgtgag aacaccaagt ttgttaccaa gatgtacgac ctgctgctgc    1140

tcaagtgtgc ccgcctcttc cccgacagca acctggaggc cgtgctcaac aacgacggcc    1200

tctcgcccct catgatggct gccaagacgg gcaagattgg gatctttcag cacatcatcc    1260

ggcgggaggt gacggatgag gacacacggc acctgtcccg caagttcaag gactgggcct    1320

atgggccagt gtattcctcg ctttatgacc tctcctccct ggacacgtgt ggggaagagg 1380
cctccgtgct ggagatcctg gtgtacaaca gcaagattga gaaccgccac gagatgctgg 1440
ctgtggagcc catcaatgaa ctgctgcggg acaagtggcg caagttcggg gccgtctcct 1500
tctacatcaa cgtggtctcc tacctgtgtg ccatggtcat cttcactctc accgcctact 1560
accagccgct ggagggcaca ccgccgtacc cttaccgcac cacggtggac tacctgcggc 1620
tggctggcga ggtcattacg ctcttcactg gggtcctgtt cttcttcacc aacatcaaag 1680
acttgttcat gaagaaatgc cctggagtga attctctctt cattgatggc tccttccagc 1740
tgctctactt catctactct gtcctggtga tcgtctcagc agccctctac ctggcaggga 1800
tcgaggccta cctggccgtg atggtctttg ccctggtcct gggctggatg aatgcccttt 1860
acttcacccg tgggctgaag ctgacgggga cctatagcat catgatccag aagattctct 1920
tcaaggacct tttccgattc ctgctcgtct acttgctctt catgatcggc tacgcttcag 1980
ccctggtctc cctcctgaac ccgtgtgcca acatgaaggt gtgcaatgag gaccagacca 2040
actgcacagt gcccacttac ccctcgtgcc gtgacagcga gaccttcagc accttcctcc 2100
tggacctgtt taagctgacc atcggcatgg gcgacctgga gatgctgagc agcaccaagt 2160
accccgtggt cttcatcatc ctgctggtga cctacatcat cctcaccttt gtgctgctcc 2220
tcaacatgct cattgccctc atgggcgaga cagtgggcca ggtctccaag gagagcaagc 2280
acatctggaa gctgcagtgg gccaccacca tcctggacat tgagcgctcc ttccccgtat 2340
tcctgaggaa ggccttccgc tctggggaga tggtcaccgt gggcaagagc tcggacggca 2400
ctcctgaccg caggtggtgc ttcagggtgg atgaggtgaa ctggtctcac tggaaccaga 2460
acttgggcat catcaacgag gacccgggca agaatgagac ctaccagtat tatggcttct 2520
cgcataccgt gggccgcctc cgcagggatc gctggtcctc ggtggtaccc cgcgtggtgg 2580
aactgaacaa gaactcgaac ccggacgagg tggtggtgcc tctggacagc atggggaacc 2640
cccgctgcga tggccaccag cagggttacc cccgcaagtg gaggactgat gacgccccgc 2700
tctagggact gcagcccagc cccagcttct ctgcccactc atttctagtc cagccgcatt 2760
tcagcagtgc cttctggggt gtccccccac accctgcttt ggccccagag gcgagggacc 2820
agtggaggtg ccagggaggc cccaggaccc tgtggtcccc tggctctgcc tccccaccct 2880
ggggtggggg ctcccggcca cctgtcttgc tcctatggag tcacataagc caacgccaga 2940
gcccctccac ctcaggcccc agcccctgcc tctccattat ttatttgctc tgctctcagg 3000
aagcgacgtg acccctgccc cagctggaac ctggcagagg ccttaggacc ccgttccaag 3060
tgcactgccc ggccaagccc cagcctcagc ctgcgcctga gctgcatgcg ccaccatttt 3120
tggcagcgtg gcagctttgc aaggggctgg ggccctcggc gtggggccat gccttctgtg 3180
tgttctgtag tgtctgggat ttgccggtgc tcaataaatg tttattcatt gacggtgaaa 3240

Glu Phe Ile Asn Ser Pro Phe Arg Asp Ile Tyr Tyr Arg Gly Gln Thr
225                 230                 235                 240

Ala Leu His Ile Ala Ile Glu Arg Arg Cys Lys His Tyr Val Glu Leu
                245                 250                 255

Leu Val Ala Gln Gly Ala Asp Val His Ala Gln Ala Arg Gly Arg Phe
            260                 265                 270

Phe Gln Pro Lys Asp Glu Gly Gly Tyr Phe Tyr Phe Gly Glu Leu Pro
        275                 280                 285

Leu Ser Leu Ala Ala Cys Thr Asn Gln Pro His Ile Val Asn Tyr Leu
    290                 295                 300

Thr Glu Asn Pro His Lys Lys Ala Asp Met Arg Arg Gln Asp Ser Arg
305                 310                 315                 320

Gly Asn Thr Val Leu His Ala Leu Val Ala Ile Ala Asp Asn Thr Arg
                325                 330                 335

Glu Asn Thr Lys Phe Val Thr Lys Met Tyr Asp Leu Leu Leu Leu Lys
            340                 345                 350

Cys Ala Arg Leu Phe Pro Asp Ser Asn Leu Glu Ala Val Leu Asn Asn
        355                 360                 365

Asp Gly Leu Ser Pro Leu Met Met Ala Ala Lys Thr Gly Lys Ile Gly
    370                 375                 380

Ile Phe Gln His Ile Ile Arg Arg Glu Val Thr Asp Glu Asp Thr Arg
385                 390                 395                 400

His Leu Ser Arg Lys Phe Lys Asp Trp Ala Tyr Gly Pro Val Tyr Ser
                405                 410                 415

Ser Leu Tyr Asp Leu Ser Ser Leu Asp Thr Cys Gly Glu Glu Ala Ser
            420                 425                 430

Val Leu Glu Ile Leu Val Tyr Asn Ser Lys Ile Glu Asn Arg His Glu
        435                 440                 445


    450                 455                 460


465                 470                 475                 480


485 490 495


500 505 510


515 520 525


530 535 540


545 550 555 560


565 570 575


580 585 590


595 600 605


610 615 620


625 630 635 640


645 650 655


660 665 670


675 680 685


690 695 700


705 710 715 720


725 730 735


            740                 745                 750


        755                 760                 765


    770                 775                 780


785                 790                 795                 800


                805                 810                 815


            820                 825                 830


        835                 840                 845


    850                 855                 860


865                 870

## Claims

1. A pharmaceutical composition comprising
(a) a polynucleotide comprising or consisting of a nucleotide sequence encoding a polypeptide with the amino acid sequence as set forth in SEQ ID NO: 2;
(b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity;
(c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or
(d) a polypeptide encoded by the polynucleotide of any one of (a) to (c).

2. Use of
(a) a polynucleotide comprising or consisting of a nucleotide sequence encoding a polypeptide with the amino acid sequence as set forth in SEQ ID NO: 2;
(b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity;
(c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or
(d) a polypeptide encoded by the polynucleotide of any one of (a) to (c) for the preparation of a pharmaceutical composition for the treatment or prevention of an ischemic condition and/or for the treatment of a patient undergoing tissue engineering.

3. The use of claim 2, wherein said treatment or prevention is effected by the stimulation of arteriogenesis.

4. The use of claim 2 or 3, wherein said pharmaceutical composition is to delivered to the organ or body part affected by said ischemic condition or undergoing tissue engineering.

5. The pharmaceutical composition of claim 1, 3 or 4 or the use of any one of claims 2 to 4, wherein said nucleotide sequence has the sequence set forth in SEQ ID NO: 1.

6. The pharmaceutical composition of claim 1 or 3 to 5 or the use of any of claims 2 to 5, wherein said nucleotide sequence is comprised in a vector.

7. The pharmaceutical composition or use of claim 6, wherein said polynucleotide or said vector is comprised in a liposome.

8. The pharmaceutical composition or use of claim 6, wherein said vector is an adenoviral vector, adeno-associated viral vector or lentiviral vector.

9. The pharmaceutical composition of claim 1 or 3 to 6 or the use of any of claims 2 to 6, wherein said nucleotide sequence has been transfected into a cell obtained from a patient to be treated.

10. The use of any one of claims 2 to 9, wherein said ischemic condition is a consequence of arterial injury, arterial degeneration, arterial occlusion or arterial re-occlusion.

11. The use of any one of claims 2 to 10, wherein said ischemic condition is selected from atherosclerosis, angina pectoris, infarct, heart attack, stroke, renal infarct, hypertension, wounds, peripheral ischemic vascular disease, bone fracture and abortus imminens.

12. The use of any one of claims 2 to 11, wherein the affected organs or body parts are selected from heart, brain, kidneys, limbs and blood vessels.

13. The pharmaceutical composition of any one of claim 1 or 3 to 9 or the use of any one of claims 2 to 12, wherein said pharmaceutical composition comprises said nucleotide sequence and/or said polypeptide as the sole pharmaceutically active agent(s).

14. The pharmaceutical composition of any one of claim 1 or 3 to 9 or the use of any one of claims 2 to 12, wherein said pharmaceutical composition additionally comprises one or more angiogenic growth factor(s).

15. The pharmaceutical composition or the use of claim 14, wherein said angiogenic growth factor is selected from the group consisting of FGF-2 (bFGF), FGF-4, MCP-1, PDGF and VEGF.

16. An in vitro or ex vivo method of ensuring blood supply in the course of tissue engineering, wherein the method comprises bringing the tissue into contact with
(a) a polynucleotide comprising or consisting of a nucleotide sequence encoding a polypeptide with the amino acid sequence as set forth in SEQ ID NO: 2;
(b) a polynucleotide comprising or consisting of a nucleotide sequence which is a fragment of a nucleotide sequence encoding the polypeptide with the amino acid sequence as set forth in SEQ ID NO: 2, wherein the polypeptide encoded by said polynucleotide has arteriogenic activity;
(c) a polynucleotide comprising or consisting of a nucleotide sequence encoding a polypeptide, wherein said polypeptide exhibits at least 70% sequence identity with the sequence of the polypeptide encoded by the polynucleotide of (a) or (b) over the entire length, and wherein said polypeptide has arteriogenic activity; and/or
(d) a polypeptide encoded by the polynucleotide of any one of (a) to (c).

17. A method of identifying modulators of the biological activity of the polynucleotide or the polypeptide as defined in claim 1, comprising the following steps:
(a) bringing into contact said polynucleotide or polypeptide with a test compound; and
(b) comparing the biological activity in the presence of said test compound with the biological activity in the absence of said test compound,
wherein the biological activity in the presence of a modulator and in the absence thereof differ, wherein said biological activity is the expression of said polynucleotide and/or the stimulation of arteriogenesis.

18. The method of claim 17, wherein said modulator is an activator.

19. The method of claim 17, wherein said modulator is an inhibitor.

20. An antibody identified or obtainable by the method of any one of claims 17 to 19.

21. Use of an activator of a polypeptide, said polypeptide being defined in claim 1, for the preparation of a pharmaceutical composition for the treatment or prevention of an ischemic condition and/or for the treatment of a patient undergoing tissue engineering.

22. The use of claim 21, wherein said activator is a cation channel opener.

23. The use of claim 21 or 22, wherein said activator is an epoxy-eicosatrienoic acid, preferably an 11,12-epoxy-eicosatrienoic acid, more preferred 11,12-epoxy-eicosa-5,8,14-trienoic acid.

24. Use of an inhibitor of a polypeptide, said polypeptide being defined in claim 1, for the preparation of a pharmaceutical composition for the treatment or prevention of restenosis following stent implementation.

25. The use of claim 24, wherein said pharmaceutical composition comprises a stent.

26. A pharmaceutical composition comprising or consisting of a stent, an inhibitor of a polypeptide, the polypeptide being defined in claim 1, and optionally a pharmaceutically acceptable carrier.

27. The use of claim 24 or 25 or the pharmaceutical composition of claim 26,
wherein said inhibitor is selected from the group consisting of
(a) an siRNA, an antisense nucleic acid, a ribozyme binding specifically the polynucleotide defined in claim 1;
(b) an antibody, aptamer, and a fragment or derivative thereof binding specifically the polypeptide defined in claim 1.
